# EUROPÄISCHE PATENTANMELDUNG

(11) **EP 2 417 994 A1**
(43) Veröffentlichungstag der Anmeldung: **15.02.2012**
(21) Anmeldenummer: 11171514.0
(22) Anmeldetag: 27.06.2011
(51) Int. Cl.: A61M 16/06

(54) **Beatmungsmaske für die gleichzeitige Mund- und Nasenbeatmung**

(30) Priorität: 12.08.2010 DE 202010011334 U
(71) Anmelder: Airtec Beatmungshilfen GmbH & Co KG, 45468 Mülheim an der Ruhr (DE)
(72) Erfinder: Menzel, Stefan, 45239 Essen (DE)
(74) Vertreter: Cohausz & Florack

(57) **Zusammenfassung**

Die Erfindung betrifft ein Verfahren zur Herstellung einer Beatmungsmaske (1) für die gleichzeitige Mund- und Nasenbeatmung, umfassend Abformen eines Teils des Gesichts eines Patienten, Formen eines Maskenelements (2), derart, dass dessen Innenoberfläche zumindest abschnittsweise der Kontur des abgeformten Teils des Gesichts entspricht, wobei das Maskenelement (2) einen ersten an die Gesichtsanatomie des Patienten individuell angepassten Dichtabschnitt (5) erhält, dessen Oberfläche der Kontur der Nasenunterseite des Gesichts entspricht, wobei das Maskenelement (2) einen zweiten an die Gesichtsanatomie des Patienten individuell angepassten Dichtabschnitt (7) erhält, dessen Oberfläche der Kontur des Teils des Gesichts zwischen Oberlippe und Nasolabialwinkel entspricht, und Verbinden des ersten Dichtabschnitts (5) und des zweiten Dichtabschnitts (7) miteinander. Ferner betrifft die Erfindung eine entsprechende Beatmungsmaske (1). Dadurch wird ein optimaler Sitz gewährleistet.

## Beschreibung

Die Erfindung betrifft eine Beatmungsmaske für die gleichzeitige Mund- und Nasenbeatmung, mit einem flexiblen, an die Gesichtsanatomie des Patienten individuell angepassten Maskenelement, das eine Innenseite hat, die dem Gesicht zugewandt wird, das eine Außenseite hat, die vom Gesicht abgewandt wird, das als Vollkörper aus einem elastischen Material ausgebildet ist, das so geformt ist, dass es den Bereich der Mund- und Nasenöffnungen des Patienten umschließen kann und das eine die Innenseite des Maskenelements mit der Außenseite des Maskenelements verbindende Außenöffnung aufweist.

Beatmungsmasken für die gleichzeitige Mund- und Nasenbeatmung (auch Mund-Nasen-Beatmungsmasken genannt) der vorstehend beschriebenen Art sind in der Medizintechnik seit Jahren bekannt. Neben dem Einsatz in der Intensivmedizin kommen sie insbesondere bei der Klinik- und Heimbehandlung chronischer respiratorischer Erkrankungen zum Einsatz, bei denen der Patient beispielsweise nachts auf eine Beatmungshilfe angewiesen ist. Im Vordergrund steht hierbei die Behandlung der Schlafapnoe und der Heimbeatmung, z.B. bei COPD-Patienten. Beatmungsmasken der eingangs genannten Art werden aber auch bei MS-Patienten eingesetzt.

Eine aus der Praxis bekannte, individuell angepasste Mund-Nasen-Beatmungsmaske umfasst einen Silikonvollkörper als flexibles Maskenelement, welcher derart geformt ist, dass er über seine Innenseite unmittelbar an Mund und Nase des Patienten anliegt. Bei dieser Maske sind darüber hinaus an der Innenseite des Silikonvollkörpers zwei einen Beatmungsschlitz begrenzende Stege angeformt, die beim Tragen der Maske in den Mund des Patienten eingeführt werden. Ferner umfasst die vorstehend beschriebene Maske ein oberhalb der Nase und unterhalb des Mundes angeordnetes Kunststoffversteifungselement, welches außenseitig in den Silikonvollkörper der Beatmungsmaske eingelassen ist und der Befestigung von Haltebändern dient. Solche Masken weisen hinsichtlich des Tragekomforts einige gravierende Nachteile auf. So durchströmt die Beatmungsluft den Maskenvollkörper aufgrund der direkten Anlage an Mund und Nase des Patienten mit vergleichsweise hoher Geschwindigkeit, was bei der Nasenatmung langfristig zu einer Schleimhautaustrocknung führt. Aufgrund der massiven Ausführung des Maskenkörpers kommt es ferner immer wieder zu Druckstellen an Nasenwurzel und Nasenrücken. Zudem führt die unzureichende Flexibilität des Maskenkörpers bei einseitiger Belastung, wie sie beim Schlafen des Patienten auf der Seite vorkommt, zu Undichtigkeiten, denen einerseits nur durch eine Beatmungsdruckerhöhung oder durch ein Anziehen der Haltebänder, was seinerseits wieder zu stärker ausgebildeten Druckstellen führt, begegnet werden kann. Schließlich kann es durch die Auflage der Maske im Bereich der Frontzähne zu dauerhaften Schäden und/oder Druckschmerzen an den Zahnhälsen kommen. Ein weiteres Problem ist die Versorgung von Patienten mit Platzangst, die zu eng anliegende Masken nicht tolerieren.

Aus der DE 10 2006 039 448 A1 ist eine Mund-Nasen-Beatmungsmaske bekannt, die ein inneres flexibles Maskenelement in Form eines aus einem elastischen Material bestehenden Vollkörpers sowie ein außen darauf aufliegendes Versteifungselement umfasst. Eine umlaufende Dichtlippe umschließt den gesamten von Mund und Nase eingenommenen Bereich und dichtet damit den Mund-Nasen-Bereich durch Anlage am Gesicht des Patienten gegenüber der Umgebung ab. Die umlaufende Dichtlippe ist ebenso wie der Vollkörper des Maskenelements an die Gesichtsanatomie des Patienten individuell angepasst und bewirkt somit einen relativ angenehmen Sitz der Beatmungsmaske bei gleichzeitig relativ guter Abdichtung.

Problematisch bei der Verwendung von Beatmungsmasken jeglicher Art ist jedoch, dass bei einem relativ hohen Innendruck in der Maske das Gesicht des Patienten nachgibt, was dazu führt, dass sich die Gesichtskontur gegenüber der bei der individuellen Anpassung des Maskenelements zugrunde gelegten zwangsläufig verändert. Diese Veränderung wird zusätzlich noch dadurch verstärkt, dass sich im Schlaf die Gesichtsmuskulatur entspannt. Auch ein schwaches Bindegewebe des Patienten verstärkt den zuvor beschriebenen Effekt. Dies kann unter Umständen eine Verschlechterung des Sitzes der Beatmungsmaske im bestimmungsgemäß angelegten Zustand haben, was wiederum die Abdichtung negativ beeinflussen kann und unter Umständen sogar Druckstellen hervorrufen kann.

Es ist daher die Aufgabe der vorliegenden Erfindung, eine Beatmungsmaske der zuvor beschriebenen Art dahingehend weiterzubilden, dass auch unter den zuvor beschriebenen besonderen Bedingungen ein optimaler Sitz gewährleistet ist.

Die zuvor hergeleitete und aufgezeigte Aufgabe wird gemäß einer ersten Lehre der vorliegenden Erfindung bei einer Beatmungsmaske für die gleichzeitige Mund- und Nasenbeatmung, mit einem flexiblen, an die Gesichtsanatomie des Patienten individuell angepassten Maskenelement, das eine Innenseite hat, die dem Gesicht zugewandt wird, das eine Außenseite hat, die vom Gesicht abgewandt wird, das als Vollkörper aus einem elastischen Material ausgebildet ist, das so geformt ist, dass es den Bereich der Mund- und Nasenöffnungen des Patienten umschließen kann und das eine die Innenseite des Maskenelements mit der Außenseite des Maskenelements verbindende Außenöffnung aufweist, dadurch gelöst, dass das Maskenelement einen ersten an die Gesichtsanatomie des Patienten individuell angepassten Dichtabschnitt mit einer Dichtlippe aufweist, die so geformt ist, dass sie eine Mundbeatmungsöffnung bildet, sowie einen zweiten an die Gesichtsanatomie des Patienten individuell angepassten Dichtabschnitt mit mindestens einer Dichtlippe, die so geformt ist, dass sie eine separate Nasenbeatmungsöffnung bildet, wobei der erste Dichtabschnitt und der zweite Dichtabschnitt miteinander verbunden sind.

Es wird also eine Beatmungsmaske geschaffen, die ein Maskenelement aufweist, welches eine Dichtlippe hat, die im bestimmungsgemäß angelegten Zustand, wenn die Maske also mit dem Gesicht des Patienten verbunden ist, den Bereich um den Mund umgibt, insbesondere vollständig umgibt, und durch die individuelle Anpassung an die Gesichtsanatomie des Patienten diesen Bereich stützt. Gleichzeitig weist das Maskenelement mindestens eine weitere Dichtlippe, vorzugsweise zwei Dichtlippen, auf, die im Bereich um eine oder beide Nasenöffnungen ebenfalls das Gewebe des Patienten unterstützen. Indem die um die Mundöffnung des Patienten anlegbare Dichtlippe Teil eines ersten Dichtabschnitts des Maskenelements ist, ferner die um die jeweilige Nasenöffnung anlegbare Dichtlippe Teil eines separaten Dichtabschnitts des Maskenelements ist und die beiden Dichtabschnitte miteinander verbunden sind, wird eine zusätzliche Stabilität der Dichtabschnitte erreicht, da diese sich gegenseitig halten. Ein erhöhter Maskeninnendruck, ein schwaches Bindegewebe und/oder eine im Schlaf entspannte Gesichtsmuskulatur können, da die beiden Dichtabschnitte im bestimmungsgemäß angelegten Zustand der Beatmungsmaske an diesen Stellen mit dem Gesicht in Kontakt sind, keine nennenswerte Veränderung der Gesichtskontur mehr hervorrufen. So halten die Dichtabschnitte zumindest über die Dichtlippen die am meisten betroffenen Stellen des Gesichts des Patienten immer in einer optimalen Position. Dies betrifft insbesondere den Bereich des Gesichts zwischen der Oberlippe und dem Nasolabialwinkel, also der Stelle, an der der im Wesentlichen horizontal verlaufende Nasensteg auf die im Wesentlichen vertikal verlaufende Gesichtsfläche zwischen Nase und Mund, die das Philtrum enthält, trifft. In diesem Bereich verläuft dann ein stegförmiger Abschnitt des Maskenelements, der sich vorzugsweise über die gesamte Breite des Mundes des Patienten bzw. der von der ersten Dichtlippe geformten Mundbeatmungsöffnung erstreckt.

Wie zuvor beschrieben ist neben der die Mundbeatmungsöffnung bildenden Dichtlippe mindestens eine weitere Dichtlippe vorgesehen, die so geformt ist, dass sie eine separate Nasenbeatmungsöffnung bildet. Diesbezüglich sind zwei alternative Ausgestaltungen denkbar. So kann einerseits vorgesehen sein, dass der zweite Dichtabschnitt nur eine Dichtlippe aufweist, die so geformt ist, dass sie die einzige vorhandene Nasenbeatmungsöffnung bildet. In diesem Fall würde die Dichtlippe dann vorzugsweise um den gesamten Bereich herum angeordnet sein, den die beiden Nasenöffnungen des Patienten einnehmen. Andererseits kann aber, was bevorzugt ist, der zweite Dichtabschnitt zwei Dichtlippen aufweisen, die so geformt sind, dass jede der Dichtlippen jeweils eine separate Nasenbeatmungsöffnung bildet. In letzterem Fall wäre dann je Nasenöffnung des Patienten eine separate Dichtlippe vorgesehen. Auf diese Weise ist eine noch bessere Abdichtung und damit optimale Beatmung durch die Nasenöffnungen des Patienten gewährleistet. Außerdem ist bei Vorhandensein von zwei Dichtlippen, die je einer Nasenöffnung zugeordnet sind, auch eine bessere Fixierung des Maskenelements relativ zur Gesichtsoberfläche des Patienten möglich. Ferner wird, insbesondere wenn die zwei Dichtlippen des zweiten Dichtabschnitts von zur Innenseite des Maskenelements hervorstehenden Auskragungen gebildet werden, die in die Nasenöffnungen des Patienten eingeführt werden können, die Stabilität des Maskenelements und insbesondere der beiden Dichtabschnitte erhöht und noch besser verhindert, dass sich die beiden Dichtabschnitte relativ zueinander bewegen können. Dies wiederum hat zur Folge, dass sich die Gesichtsanatomie des Patienten auch bei einem relativ hohen Innendruck in der Maske, bei einem schwachen Bindegewebe und/oder bei im Schlaf entspannter Gesichtsmuskulatur nicht mehr nennenswert verändern kann.

Gemäß einer weiteren Ausgestaltung der erfindungsgemäßen Beatmungsmaske besteht der gesamte Bereich des Maskenelements zwischen der Dichtlippe des ersten Dichtabschnitts und der mindestens einen Dichtlippe des zweiten Dichtabschnitts, insbesondere der gesamte Bereich zwischen der äußeren Kante der Dichtlippe des ersten Dichtabschnitts und der äußeren Kante der mindestens einen Dichtlippe des zweiten Dichtabschnitts, aus dem elastischen Material. Dies erhöht den Tragekomfort für den Patienten und vermindert das Risiko von Druckstellen, auch und gerade in dem Fall, dass der gesamte Bereich des Maskenelements zwischen der Dichtlippe des ersten Dichtabschnitts und der mindestens einen Dichtlippe des zweiten Dichtabschnitts, insbesondere der gesamte Bereich zwischen der äußeren Kante der Dichtlippe des ersten Dichtabschnitts und der äußeren Kante der mindestens einen Dichtlippe des zweiten Dichtabschnitts, so geformt ist, dass er mit dem Gesicht des Patienten, gemeint ist die Gesichtsoberfläche, in Anlage bringbar ist.

Gemäß noch einer weiteren Ausgestaltung der erfindungsgemäßen Beatmungsmaske besteht der gesamte Bereich des Maskenelements zwischen den beiden Dichtlippen des zweiten Dichtabschnitts, insbesondere der gesamte Bereich zwischen den äußeren Kanten der beiden Dichtlippen des zweiten Dichtabschnitts, aus dem elastischen Material. Auch dieser Bereich, in welchem beim Patienten der Nasensteg verläuft, kann zu Druckstellen führen, wenn der besagte Bereich so geformt ist, dass er mit dem Gesicht des Patienten in Anlage bringbar ist.

Ist das elastische Material in den Bereichen zwischen den genannten Dichtlippen vorgesehen und ist im bestimmungsgemäß angelegten Zustand der Beatmungsmaske das elastische Material mit der Gesichtsoberfläche des Patienten an diesen Stellen in Kontakt, so wird der Effekt noch verstärkt, wonach auch bei einem hohen Maskeninnendruck, einem schwachen Bindegewebe und/oder im Schlaf entspannter Gesichtsmuskulatur die Gesichtsanatomie bzw. -kontur weitestgehend erhalten bleibt.

An der Innenseite des Maskenelements ist gemäß noch einer weiteren Ausgestaltung der erfindungsgemäßen Beatmungsmaske ein Hohlraum ausgebildet, der mit Ausnahme der Außenöffnung sowie der Mund- und Nasenbeatmungsöffnungen von dem elastischen Material umschlossen ist. Ein solcher Hohlraum gewährleistet, dass zwar die besagten Dichtlippen, nicht aber das übrige Maskenelement unmittelbar am Gesicht des Patienten anliegt, wodurch gewährleistet ist, dass die bei der Mundund Nasenbeatmung in die Maske einströmende Luft nicht unmittelbar mit hoher Geschwindigkeit in die Mund- und Nasenöffnungen einströmt, sondern zunächst in einem umschlossenen freien Beatmungsvolumen verwirbelt, erwärmt und befeuchtet wird, wodurch einem Austrocknen der Atemwege wirksam begegnet wird. Im Gegensatz zu konfektionierten Beatmungsmasken wird das freie Beatmungsvolumen durch die Anpassung an die Gesichtsanatomie aber minimal gehalten, um eine möglichst effiziente Beatmung zu gewährleisten.

Gemäß noch einer weiteren Ausgestaltung der erfindungsgemäßen Beatmungsmaske ist eine Vertiefung zur Aufnahme der Nase des Patienten vorgesehen, die aus dem elastischen Material besteht und in die die mindestens eine Nasenbeatmungsöffnung führt. Eine solche Vertiefung führt zu einem noch weiter verbesserten Sitz der Beatmungsmaske im bestimmungsgemäß angelegten Zustand. Dabei ist es denkbar, dass der zweite Dichtabschnitt, in Richtung von der Innenseite zur Außenseite des Maskenelements, bis zum tiefsten Punkt der Vertiefung reicht, in welchem also beim Patienten die Nasenspitze angeordnet ist, wenn die Maske aufgesetzt ist. Auf diese Weise ist es möglich, dass die gesamte Nasenunterseite mit einem Teil des Maskenelements in Kontakt kommt, was den Sitz der Maske und die Beatmung weiter optimiert.

Der erste Dichtabschnitt und/oder der zweite Dichtabschnitt ist gemäß wiederum einer weiteren Ausgestaltung der erfindungsgemäßen Beatmungsmaske einstückig mit dem übrigen Teil des Maskenelements ausgeführt. Ein solches Maskenelement ist einerseits auf besonders einfache Weise herstellbar, führt andererseits aber auch zu einem optimalen Tragekomfort und letztlich zu einer optimalen Beatmung, da Verbindungsstellen, die zu ungünstigen Kanten führen können und die sich im Laufe der Zeit lösen können, vermieden werden. Durch eine einstückige Ausführung können auch optimale Übergänge zwischen dem jeweiligen Dichtabschnitt und dem übrigen Teil des Maskenelements geschaffen werden.

Um eine optimale Stabilität des gesamten Maskenelements und damit der Beatmungsmaske zu gewährleisten, ist gemäß einer weiteren Ausgestaltung der erfindungsgemäßen Beatmungsmaske vorgesehen, dass der erste Dichtabschnitt und/oder der zweite Dichtabschnitt flexibler als der übrige Teil des Maskenelements ausgeführt ist, mit anderen Worten also der übrige Teil des Maskenelements relativ steif ausgebildet ist. Dies führt zu einem dichten Sitz der Beatmungsmaske, insbesondere auch bei einer einseitigen Belastung, wie sie zum Beispiel beim Schlafen des Patienten auf der Seite auftritt. Auch werden dadurch die beiden Dichtabschnitte in jeder Lage des Patienten an Ort und Stelle gehalten, wodurch letztlich auch das Risiko der Veränderung der Gesichtsanatomie des Patienten verhindert wird, wenn beispielsweise ein relativ hoher Maskeninnendruck herrscht, der Patient ein schwaches Bindegewinde hat und/oder die Muskulatur im Schlaf entspannt.

Die im vorangehenden Absatz genannten Vorteile werden weiter dadurch verstärkt, wenn gemäß einer weiteren Ausgestaltung der erfindungsgemäßen Beatmungsmaske mindestens ein mit dem Maskenelement verbundenes Versteifungselement vorgesehen ist, das an der Außenseite des Maskenelements anliegt. Alternativ oder zusätzlich kann das mindestens eine Versteifungselement auch in das Maskenelement zumindest teilweise eingebettet sein. An einem solchen Versteifungselement lassen sich dann ohne weiteres auch Befestigungsmöglichkeiten vorsehen, um die Beatmungsmaske am Kopf des Patienten zu fixieren.

Die Aufgabe wird ferner gemäß einer zweiten Lehre der vorliegenden Erfindung bei einem Verfahren zur Herstellung einer Beatmungsmaske für die gleichzeitige Mund- und Nasenbeatmung, insbesondere einer Beatmungsmaske nach einem der vorangehenden Ansprüche, wobei ein Teil des Gesichts eines Patienten abgeformt wird und ein Maskenelement geformt wird, dessen Innenoberfläche zumindest abschnittsweise der Kontur des abgeformten Teils des Gesichts entspricht, dadurch gelöst, dass das Maskenelement einen ersten an die Gesichtsanatomie des Patienten individuell angepassten Dichtabschnitt erhält, dessen Oberfläche der Kontur der Nasenunterseite des Gesichts entspricht, sowie einen zweiten an die Gesichtsanatomie des Patienten individuell angepassten Dichtabschnitt, dessen Oberfläche der Kontur des Teils des Gesichts zwischen Oberlippe und Nasolabialwinkel entspricht, wobei der erste Dichtabschnitt und der zweite Dichtabschnitt miteinander verbunden werden. Dabei ist bevorzugt, wenn der erste Dichtabschnitt eine Oberfläche erhält, die der Kontur der gesamten Nasenunterseite vom Nasolabialwinkel bis zur Nasenspitze entspricht.

Wie bereits bei der Beschreibung der Beatmungsmaske erläutert, führt auch das zuvor definierte Herstellungsverfahren dazu, dass im bestimmungsgemäß angelegten Zustand einer solchermaßen hergestellten Beatmungsmaske die Gesichtsanatomie des Patienten weitgehend erhalten bleibt, auch wenn ein hoher Innendruck in der Maske vorliegt, der Patient ein schwaches Bindegewebe hat und/oder die Gesichtsmuskulatur im Schlaf entspannt ist.

Es gibt nun eine Vielzahl von Möglichkeiten, die erfindungsgemäße Beatmungsmaske und das erfindungsgemäße Herstellungsverfahren auszugestalten und weiterzubilden. Hierzu sei einerseits verwiesen auf die dem Patentanspruch 1 nachgeordneten Patentansprüche, andererseits auf die Beschreibung eines Ausführungsbeispiels in Verbindung mit der Zeichnung.

In der Zeichnung zeigt:
- Fig. 1: eine Innenansicht einer erfindungsgemäßen Beatmungsmaske und
- Fig. 2: eine seitliche Schnittansicht der Beatmungsmaske aus Fig. 1.

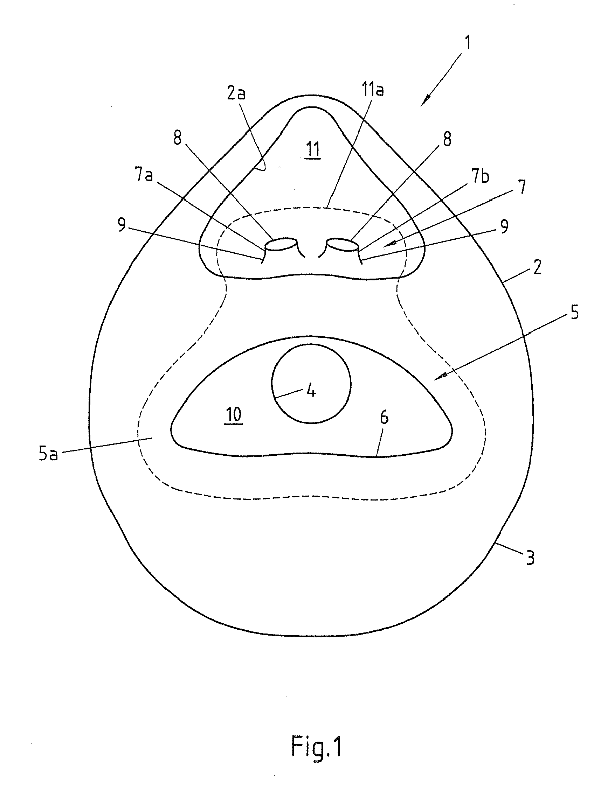

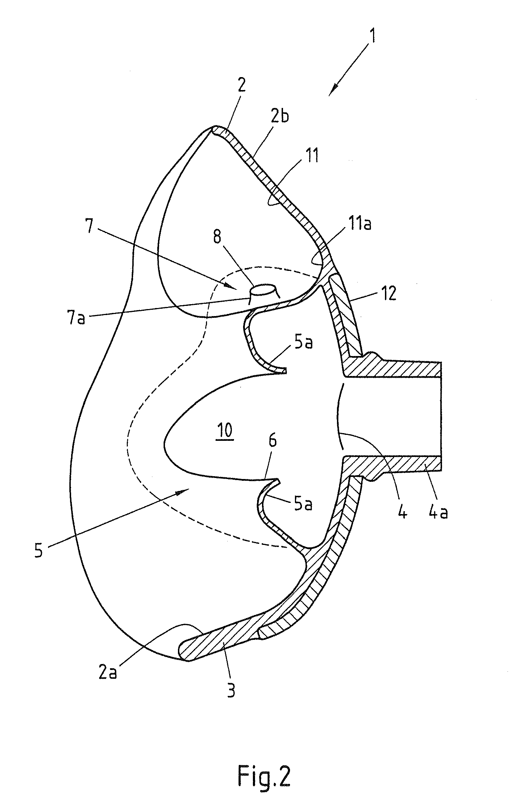

In den Figuren 1 und 2 ist eine Beatmungsmaske 1 für die gleichzeitige Mund- und Nasenbeatmung dargestellt, die ein flexibles, an die Gesichtsanatomie bzw. -kontur des Patienten individuell angepasstes Maskenelement 2 und ein zusätzliches Versteifungselement 12 aufweist.

Das Maskenelement 2 hat eine Innenseite 2a und eine Außenseite 2b und ist als Vollkörper 3 aus einem elastischen Material ausgebildet.

Das Maskenelement 2 ist so geformt, dass es den Bereich der Mund- und Nasenöffnungen des Patienten umschließen kann und eine die Innenseite 2a des Maskenelements 2 mit der Außenseite 2b verbindende Außenöffnung 4 aufweist, die über ein Anschlussstück 4a mit einem Beatmungsschlauch verbunden werden kann.

Das Maskenelement 2 weist einen ersten an die Gesichtsanatomie bzw. -kontur des Patienten individuell angepassten Dichtabschnitt 5 mit einer Dichtlippe 5a auf, die so geformt ist, dass sie eine Mundbeatmungsöffnung 6 bildet, wobei die Dichtlippe 5a um den Mund des Patienten herum an der Gesichtsoberfläche zur Anlage bringbar ist. Ferner weist das Maskenelement 2 einen zweiten an die Gesichtsanatomie bzw. -kontur des Patienten individuell angepassten Dichtabschnitt 7 mit zwei Dichtlippen 7a und 7b auf, die so geformt sind, dass sie jeweils eine separate Nasenbeatmungsöffnung 8 bilden, die mit der Nasenöffnung des Patienten in Übereinstimmung bringbar ist.

Der erste Dichtabschnitt 5 und der zweite Dichtabschnitt 7 sind durch das elastische Material des Vollkörpers 3 miteinander verbunden und halten die Gesichtsoberfläche des Patienten in einem optimalen Zustand, auch wenn der Innendruck in der Maske 1 relativ hoch ist, der Patient ein schwaches Bindegewebe hat und/oder die Gesichtsmuskeln im Schlaf entspannt sind.

Wie die Figuren 1 und 2 ferner zeigen, werden die zwei Dichtlippen 7a und 7b des zweiten Dichtabschnitts 7 von zur Innenseite 2a des Maskenelements 2 hervorstehenden Auskragungen 9 gebildet. Die Auskragungen 9 können dann in die Nasenöffnungen des Patienten eingeführt werden.

Ferner ist vorgesehen, dass der gesamte Bereich des Maskenelements 2 zwischen der Dichtlippe 5a und den Dichtlippen 7a und 7b aus dem elastischen Material besteht. Auch der gesamte Bereich zwischen den beiden Dichtlippen 7a und 7b besteht aus dem elastischen Material. Das elastische Material in den genannten Bereichen liegt dann auch im bestimmungsgemäß angelegten Zustand der Beatmungsmaske 1 an der Gesichtsoberfläche des Patienten an.

An der Innenseite des Maskenelements 2 ist ferner ein Hohlraum 10 ausgebildet, der mit Ausnahme der Außenöffnung 4 sowie der Mund- und Nasenbeatmungsöffnungen 6 und 8 von dem elastischen Material umschlossen ist.

Ferner ist eine Vertiefung 11 zur Aufnahme der Nase des Patienten vorgesehen, die aus dem elastischen Material besteht und in die die mindestens eine Nasenbeatmungsöffnung 8 führt. Wie Fig. 2 zeigt, reicht der zweite Dichtabschnitt 7, in Richtung von der Innenseite 2a zur Außenseite 2b des Maskenelements 2, bis zum tiefsten Punkt 11a der Vertiefung.

Bei der dargestellten Beatmungsmaske 1 ist sowohl der erste Dichtabschnitt 5 als auch der zweite Dichtabschnitt 7 einstückig mit dem übrigen Teil des Maskenelements 2 ausgeführt. Dabei ist der erste Dichtabschnitt 5 und der zweite Dichtabschnitt 7 flexibler als das übrige Maskenelement 2.

Das erwähnte Versteifungselement 12 ist im vorliegenden Fall so ausgebildet, dass es in Form einer gebogenen Schale außen am Maskenelement 2 anliegt. Anstelle eines solchen einstückigen Versteifungselements 12 ist aber auch denkbar, mehrere kleinere Versteifungselemente 12 vorzusehen, die dann insbesondere auch in das Maskenelement 2 zumindest teilweise eingebettet sind und bevorzugt über aus dem Maskenelement 2 nach außen hervorstehende Befestigungselemente verfügen, über die mittels entsprechender Bänder die Beatmungsmaske 1 am Kopf des Patienten fixiert werden kann.

## Patentansprüche

1. Verfahren zur Herstellung einer Beatmungsmaske (1) für die gleichzeitige Mund- und Nasenbeatmung, umfassend:
- Abformen eines Teils des Gesichts eines Patienten,
- Formen eines Maskenelements (2), derart, dass dessen Innenoberfläche zumindest abschnittsweise der Kontur des abgeformten Teils des Gesichts entspricht,
- wobei das Maskenelement (2) einen ersten an die Gesichtsanatomie des Patienten individuell angepassten Dichtabschnitt (5) erhält, dessen Oberfläche der Kontur der Nasenunterseite des Gesichts entspricht,
- wobei das Maskenelement (2) einen zweiten an die Gesichtsanatomie des Patienten individuell angepassten Dichtabschnitt (7) erhält, dessen Oberfläche der Kontur des Teils des Gesichts zwischen Oberlippe und Nasolabialwinkel entspricht, und
- Verbinden des ersten Dichtabschnitts (5) und des zweiten Dichtabschnitts (7) miteinander.

2. Verfahren nach Anspruch 1, **dadurch gekennzeichnet, dass** der erste Dichtabschnitt (5) eine Oberfläche erhält, die der Kontur der gesamten Nasenunterseite vom Nasolabialwinkel bis zur Nasenspitze entspricht.

3. Verfahren nach Anspruch 1 oder 2, **dadurchü gekennzeichnet,** dass der zweite Dichtabschnitt (7) nur eine Dichtlippe (7a,7b) aufweist, die so geformt wird, dass sie die einzige Nasenbeatmungsöffnung (8) bildet.

4. Verfahren nach Anspruch 1 oder 2, **dadurch gekennzeichnet, dass** der zweite Dichtabschnitt (7) zwei Dichtlippen (7a,7b) aufweist, die so geformt werden, dass jede der Dichtlippen (7a,7b) jeweils eine separate Nasenbeatmungsöffnung (8) bildet.

5. Verfahren nach Anspruch 4, **dadurch gekennzeichnet, dass** die zwei Dichtlippen (7a,7b) des zweiten Dichtabschnitts (7) von zur Innenseite (2a) des Maskenelements (2) hervorstehenden Auskragungen (9) gebildet werden.

6. Verfahren nach einem der vorangehenden Ansprüche, **dadurch gekennzeichnet, dass** der gesamte Bereich des Maskenelements (2) zwischen der Dichtlippe (5a) des ersten Dichtabschnitts (5) und der mindestens einen Dichtlippe (7a,7b) des zweiten Dichtabschnitts (7) aus einem elastischen Material gebildet wird.

7. Verfahren nach Anspruch 6, **dadurch gekennzeichnet, dass** der gesamte Bereich des Maskenelements (2) zwischen der Dichtlippe (5a) des ersten Dichtabschnitts (5) und der mindestens einen Dichtlippe (7a,7b) des zweiten Dichtabschnitts (7) so geformt wird, dass er mit dem Gesicht des Patienten in Anlage bringbar ist.

8. Verfahren nach einem der vorangehenden Ansprüche, **dadurch gekennzeichnet, dass** der gesamte Bereich des Maskenelements (2) zwischen den beiden Dichtlippen (7a,7b) des zweiten Dichtabschnitts (7) aus dem elastischen Material gebildet wird.

9. Verfahren nach Anspruch 8, **dadurch gekennzeichnet, dass** der gesamte Bereich des Maskenelements (2) zwischen den beiden Dichtlippen (7a,7b) des zweiten Dichtabschnitts (7) so geformt wird, dass er mit dem Gesicht des Patienten in Anlage bringbar ist.

10. Verfahren nach einem der vorangehenden Ansprüche, **dadurch gekennzeichnet, dass** an der Innenseite (2a) des Maskenelements (2) ein Hohlraum (10) ausgebildet wird, der mit Ausnahme der Außenöffnung (4) sowie der Mund- und Nasenbeatmungsöffnungen (6,8) von dem elastischen Material umschlossen ist.

11. Verfahren nach einem der vorangehenden Ansprüche, **dadurch gekennzeichnet, dass** eine Vertiefung (11) zur Aufnahme der Nase des Patienten gebildet wird, die aus dem elastischen Material besteht und in die die mindestens eine Nasenbeatmungsöffnung (8) führt, wobei insbesondere der zweite Dichtabschnitt (7), in Richtung von der Innenseite (2a) zur Außenseite (2b) des Maskenelements (2), bis zum tiefsten Punkt (11a) der Vertiefung (11) reicht.

12. Verfahren nach einem der vorangehenden Ansprüche, **dadurch gekennzeichnet, dass** der erste Dichtabschnitt (5) und/oder der zweite Dichtabschnitt (7) einstückig mit dem übrigen Teil des Maskenelements (2) ausgeführt wird.

13. Verfahren nach einem der vorangehenden Ansprüche, **dadurch gekennzeichnet, dass** der erste Dichtabschnitt (5) und/oder der zweite Dichtabschnitt (7) flexibler als der übrige Teil des Maskenelements (2) ausgeführt wird.

14. Verfahren nach einem der vorangehenden Ansprüche, **dadurch gekennzeichnet, dass** mindestens ein mit dem Maskenelement (2) verbundenes Versteifungselement (12) vorgesehen wird, das an der Außenseite (2b) des Maskenelements (2) angelegt und/oder in das Maskenelement (2) zumindest teilweise eingebettet wird.

15. Beatmungsmaske (1), hergestellt durch das Verfahren nach einem der vorangehenden Ansprüche.
